# EUROPEAN PATENT SPECIFICATION

(11) **EP 0 672 670 B1**
(45) Date of publication and mention of the grant of the patent: **14.02.1996**
(21) Application number: 95201085.8
(22) Date of filing: 07.06.1993
(51) Int. Cl.: C07D 503/18

(54) **Process for the preparation of clavulanic acid**
Verfahren zur Herstellung von Clavulansäure
Procédé de préparation de l'acide clavulanique

(30) Priority: 11.06.1992 GB 9212379; 31.10.1992 GB 9222841; 14.12.1992 GB 9226061; 17.12.1992 GB 9226282
(43) Date of publication of application: 20.09.1995
(62) Divisional of application: 93913311.2
(73) Proprietor: SMITHKLINE BEECHAM PLC, Brentford, Middlesex TW8 9EP (GB)
(72) Inventor: Cook,Michael,Allen, Worthing, West Sussex BN14 8QH (GB); Wilkins, Robert Bennett, Irvine, Ayrshire KA11 5AP (GB)
(74) Representative: Vossius, Tilman

(56) References cited:
- EP-A- 0 026 044
- EP-A- 0 562 583
- WO-A-94/22873

## Description

This invention relates to a novel process for the preparation and/or purification of clavulanic acid (I): 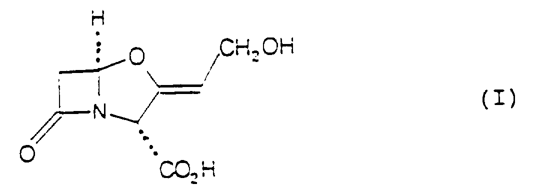 and pharmaceutically acceptable salts and esters thereof.

Clavulanic acid is normally prepared by the fermentation of a microorganism which produces clavulanic acid, such as various microorganisms belonging to various *Streptomyces* strains such as *S*. *clavuligerus* NRRL 3585, *S*. *jumoninensis* NRRL 5741, *S*. *katsurahamanus* IFO 13716 and *Streptomyces* sp. P 6621 FERM P2804 e.g. as described in JP Kokai 80-162993. The resulting aqueous broth may be subjected to conventional purification and concentration processes, for example involving filtration and chromatographic purification, such as disclosed in GB 1508977 and JP Kokai 80762993, before extraction of the aqueous solution with an organic solvent to yield a solution of crude clavulanic acid in the organic solvent.

GB 1508977 discloses inter alia that salts of clavulanic acid can be obtained by absorbing the clavulanate anion in filtered broth on to an anion exchange resin, eluting therefrom with an electrolyte, desalting the resulting solution, applying the desalted solution to a further anion exchange resin, chromatographically eluting therefrom with an electrolyte, desalting the resulting solution and thereafter removing the solvent. This process can be used to give acceptable yields of pure material but the use of resin columns involves significant investment and they can introduce limitations in large scale production operations. It would therefore be desirable to have an alternative procedure available that involved few resin utilizing stages.

GB 1543563 discloses a process for the preparation of clavulanic acid salts via precipitation of lithium clavulanate. GB 1578739 describes various amine salts of clavulanic acid as pharmaceutical compounds. EP-A-0026044 discloses the use of the tertiary-butylamine salt of clavulanic acid as a useful intermediate in the preparation of clavulanic acid. The salt has been disclosed in BE 862211, but only as a suitable ingredient for pharmaceutical formulations. PT.94.908 describes the use of tri-(lower alkyl)amine salts and the dimethylaniline salts of clavulanic acid in a purification process for clavulanic acid in which the triethylamine salt of clavulanic acid is formed and is then converted into a silyl diester of clavulanic acid. EP-A-0387178 discloses a process for the purification of clavulanic acid in which ammonia or primary, secondary or tertiary monoamines are used to form an intermediate amine salt with clavulanic acid in an impure solution.

In EP-A-0 562 583 which is not prepublished, it is suggested to extract a fermentation broth containing clavulanic acid with ethyl acetate, methyl isobutyl ketone or butanol. The organic extract is then washed with water and concentrated by evaporation to a concentration of at least 20 g/L crude clavulanic acid and to a residual water content of under 6 g/L. The obtained concentrate is further subjected to a treatment with activated charcoal to eliminate any coloured matter. The clarified extract obtained is reacted with a substituted alkylenediamine of the general formula 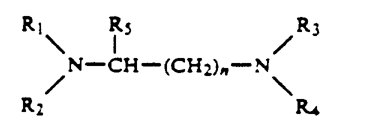 wherein the substituents R₁, R₂, R₃ and R₄ denote
a hydrogen atom,
a straight chain or a branched chain alkyl group having 1 to 8 carbon atoms,
an arylalkyl group wherein the alkyl group is a methyl or ethyl group and the aryl group is a phenyl group, which is optionally para-substituted by a methyl, methoxy, nitro or halo (chloro or bromo) group, the aryl group on the alkyl chain being in *α* - or β-position,
a hydroxyalkyl group having 2 to 4 carbon atoms,
an aminoalkyl group having 2 to 4 carbon atoms, which is optionally substituted by an N-alkyl or N,N-dialkyl group having 1 to 4 carbon atoms, or
R₁ and R₂ or R₃ and R₄ jointly independently denote a cyclic alkylene ring having 3 to 6 methylene groups, one of these groups being optionally substituted by an oxygen or a sulphur atom or by an imino group and R₅ denotes a hydrogen atom, or a methyl group and n denotes an integer from 1 to 3,
to yield alkylenediammonium diclavulanates of the formula 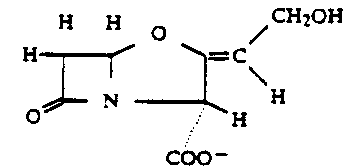 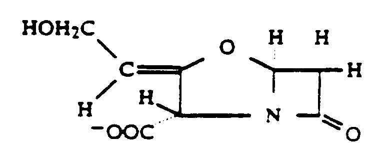 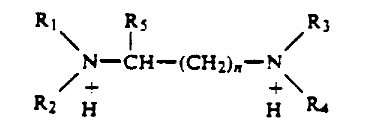 wherein the substituents have the above meanings. These salts are novel and have not yet been described in literature.

In comparison with the known method for the preparation of the clavulanic acid and pharmaceutically acceptable salts thereof as described in EP-A-0 026 044, this process excels by the preparation of the intermediate product, i.e. of the alkylenediammonium salt in high yield and purity without any need of additional purification by recrystallization, and this intermediate product can be obtained directly from the concentrate without the use of additional organic solvents, such as acetone. According to the process as described in EP-A-0 026 044 the clavulanate is precipitated with tert. butylamine, the obtained precipitate is treated with acetone, purified by reprecipitation with acetone from the isopropanol solution and then transformed in an isopropanolic solution to the potassium salt of the clavulanic acid.

The technical problem underlying the present invention is to provide a novel process for the preparation and/or purification of clavulanic acid and pharmaceutically acceptable salts and esters therof, which process occurs in high yields and is economically practicable. This technical problem is solved according to claims 1 to 9 for the designated countries AT, CH, DE, DK, ES, FR, GB, IT, LI, LU, NL and SE, and according to claims 1 to 11 for the designated countries BE, GR, IE, MC and PT respectively.
The polyamine salts of clavulanic acid are novel under Art. 54(1) and (2) EPC.

The polyamines to be used in the process of the present invention can be described by the general formula (II): 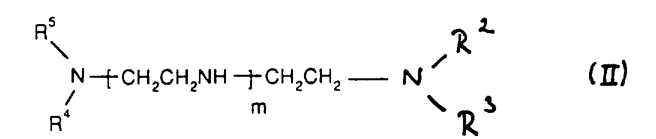 where R⁴ and R⁵ are independently hydrogen, C₁₋₆-alkyl, amino- substituted C₁₋₆-alkyl or substituted amino- substituted C₁ alkyl, and R and R³ are independently selected from hydrogen, C₁₋₆-alkyl, amino- or hydroxy- substituted C₁₋₆-alkyl or substituted amino-substituted C₁₋₆-alkyl, and m is zero or an integer of 1 to 5.

When alkyl groups or substituted alkyl groups are referred to herein unless otherwise defined herein they may suitably contain 1 to 6 carbon atoms in the alkyl group. Suitable substituents on amino groups include alkyl.

The residues R⁴ and R⁵ may suitably both be hydrogen, or one may be hydrogen and the other alkyl. R and R³ may suitably be hydrogen or alkyl.

Examples of such amines include ethylene diamine, N,N'diethylethylene diamine, N,N'-diisopropylethylenediamine and triethylene tetramine. Thus, the clavulanic acid may form a salt with one or more of the nitrogen atoms, for example as in N,N'-diisopropylethylenediamine diclavulanate.

In the process of the present invention the salt of clavulanic acid with the polyamine of the general formula (II) may be used to purify impure clavulanic acid during its preparation. Therefore the salt may be formed in a solution of clavulanic acid containing impurities, isolating the salt as a separate phase, e.g. as a solid precipitate. from the solution containing residual impurities, then reforming clavulanic acid or forming a pharmaceutically acceptable salt or ester thereof.

Conveniently the polyamine of the general formula (II) itself is contacted with impure clavulanic acid itself in solution in an organic solvent.

The above process is suitably carried out in an organic solvent, which although preferably substantially dry, for example containing less than 6 g/L, e.g. 0.25-0.6 g/L of water, may contain some water, as a solvent for the clavulanic acid and the polyamine of the general formula (II). A suitable degree of dryness may be achieved by conventional dewatering processes such as centrifugation. Water present in the solvent may be dissolved or in the form of droplets of a separate phase.

The solution of clavulanic acid in the organic solvent may be obtained by extraction of an acidified aqueous solution of clavulanic acid such as the fermentation liquor referred to above. If the initial source of the clavulanic acid is a broth resulting from fermentation of a clavulanic acid-producing microorganism, such as those mentioned above, then to obtain a solvent extract of a suitable concentration of clavulanic acid for use in the process of the present invention it may be desirable not to extract the broth itself, but to at least remove some of the suspended solids in the broth, e.g by filtration prior to extraction. It may also be desirable in addition to pre-concentrate the aqueous solution of clavulanic acid obtained in fermentation, so that for example the aqueous solution of clavulanic acid is several times more concentrated in clavulanic acid than the starting fermentation broth, for example pre-concentrated to a concentration of ca. 10- 100 mg/ml, e.g. 10 - 40 mg/ml, such as 10 - 25 g/L clavulanic acid.

Suitable pre-concentration processes include absorption of the clavulanic acid onto an anion exchange resin, followed by elution of the clavulanic acid therefrom with an aqueous solution of an electrolyte such as sodium chloride, and optionally de-salting. It is also preferred to acidify the aqueous solution, e.g. the fermentation broth or the pre-concentrated aqueous solution prior to solvent extraction, e.g. to a pH of 1 to 3, e.g. around pH 1.5 to 2.5. It is also preferred to dry or de-water the organic solvent extract prior to formation of the salt with the polyamine of the general formula (II), e.g. to less than 6g/L of water. Preferably the extraction is carried out at a temperature from 5 to 15°C.

Suitable organic solvents in which impure clavulanic acid may be contacted with the polyamine of the general formula (II) include hydrocarbon solvents such as toluene and hexane, ethers such as tetrahydrofuran, dioxan, diethyl ether, halogenated solvents such as dichloromethane and chloroform, ketones such as acetone and methyl isobutyl ketone, and esters such as ethyl acetate. Solvents which include a carbonyl group, e.g. those of the general formula (III): 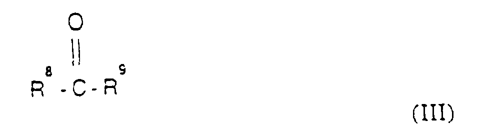 wherein R⁸ is a C₁₋₆ alkyl group or a C₁₋₆ alkoxy group and R⁹ is a C₁₋₆ alkyl group are examples of a sub-class of suitable solvents, for example ketones or alkanoate esters. The present invention also encompasses the use of mixtures of such solvents.

More suitably the organic solvent is one which can be used directly to extract the acidified aqueous solution, for example alkyl alkanoate esters, ketones and certain aliphatic alcohols, or mixtures thereof, such as ethyl acetate, methyl acetate, propyl acetate, n-butyl acetate, methyl ethyl ketone, methyl isobutyl ketone, tetrahydrofuran, butanol and mixtures of such solvents. Of these the most suitable appear to be methyl isobutyl ketone, methyl ethyl ketone, and ethyl acetate. Suitable solvent mixtures include methyl ethyl ketone/methyl isobutyl ketone and tetrahydrofuran/methyl isobutyl ketone. A preferred solvent is ethyl acetate.

Suitable solvents for the polyamine of the general formula (II) include those referred to above in which the clavulanic acid may be dissolved, or extracted, for example acetone, ethyl acetate, methyl isobutyl ketone, and methyl ethyl ketone.

It is particularly desirable to include ketones such as acetone in the solvent system, as these appear to inhibit the formation of the salt of clavulanic acid with the polyamine of the general formula (II) as an oil.

In general one equivalent of the polyamine of the general formula (II) or a slight excess thereof per mole of clavulanic acid is used to produce the salt of clavulanic acid. Solutions of clavulanic acid and polyamine of the general formula (II) may for example be mixed slowly with stirring and the mixture stirred for some time after addition is complete. The reaction between the clavulanic acid or its labile derivative is suitably carried out at a temperature below ambient, for example at 0 to 15°C, e.g. 0 to 10°C, e.g. 0 to 5°C. A suitable concentration for the clavulanic acid or its labile derivative in the solution is at least 1.0 g/L, for example in the range 1.0 to 4.0 g/L of clavulanic acid. It may be advantageous to further concentrate the solvent extract to a concentration in excess of this, e.g. greater than 20g/L.

For example in another procedure the polyamine of the general formula (II) may be introduced by mixing it into a stream of a solution of the clavulanic acid in the solvent, so that the salt is formed in the stream, either in solution or as particles or suspended droplets of the dissolved salt in suspension. The polyamine of the general formula (II) introduced in this way may be introduced as such, or may be introduced as a solution in a solvent, for example the same organic solvent as the clavulanic acid is dissolved in.

The desired salt of clavulanic acid with the polyamine of the general formula (II) may then be isolated. In this way, the salt of clavulanic acid with the polyamine of the general formula (II) is separated from most or all of the impurities. Isolation may be effected in a conventional manner, for example by centrifugation or filtration.

In an alternative isolation procedure the salt of clavulanic acid with the polyamine of the general formula (II) may be isolated from the organic solvent, if the solvent is wholly or partly immiscible with water, by contacting the solvent with water so as to extract the salt, which may be in solution or suspension, from the organic solvent and to form an aqueous solution of the salt. As salts of clavulanic acid with the polyamine of the general formula (II) are fairly soluble in water, such an aqueous solution may be very concentrated, e.g. around 20-30% by weight.

In this way the bulk of organic impurities in the organic solvent solution of clavulanic acid remain in the organic solvent whilst the clavulanic acid, in the form of its salt with the polyamine of the general formula (II), in a relatively pure state is obtained in the aqueous solution. The aqueous solution of the clavulanic acid salt so formed may be subjected to conventional further treatment, e.g. purification, or conversion into clavulanic acid or a pharmaceutically acceptable salt or ester as described below.

The pharmaceutically acceptable salts and esters of clavulanic acid prepared by the process of this invention include those described in GB 1508977 and 1508978.

Particularly suitable pharmaceutically acceptable salts include the pharmaceutically acceptable alkali and alkaline earth metal salts, for example the sodium, potassium, calcium and magnesium salts. Of these salts the sodium and potassium salts are most suitable and the potassium salt is preferred.

Suitable esters include those cleavable to provide clavulanic acid or a salt thereof, by chemical methods such as hydrogenolysis or by biological methods.

The salt of clavulanic acid with the polyamine of the general formula (II) may be converted into clavulanic acid or a pharmaceutically acceptable salt or ester thereof by for example ion-replacement in the case of the free acid or salts, or by esterification.

Ion-replacement may be performed using ion-exchange resins, for example by passing a solution of the salt through a bed of a cation exchange resin in the sodium, potassium or calcium form. Suitable cation exchange resins include Amberlite IR 120 and equivalent resins.

Alternatively ion-replacement may be effected by reaction of the protonated amine cation with a salt-precusor compound, which may be a base, for example a carbonate, bicarbonate or hydroxide of a pharmaceutically acceptable alkali or alkaline earth metal, or a salt of an organic carboxylic acid with a pharmaceutically acceptable cation such as an alkali or alkaline earth metal, for example a salt of an alkanoic acid of the general formula (IV):

R¹⁰-COOH (IV)

wherein R¹⁰ is an alkyl group containing for example from 1 to 20 carbon atoms, preferably from 1 to 8 carbon atoms. Examples of suitable salts include the acetate, propionate or ethylhexanoate salts, potassium 2-ethylhexanoate and sodium 2-ethylhexanoate being preferred. Typically the salt of clavulanic acid with the polyamine of the general formula (II) in solution may be reacted with a salt of an alkali metal with the acid of the general formula (IV) in solution or suspension in a suitable solvent, which may for example be an organic solvent, water, or a mixture of water and an organic solvent such as isopropanol. Suitably solutions of the salt of clavulanic acid with the polyamine of the general formula (II) and of the salt-precursor compound may be mixed, and the pharmaceutically acceptable salt allowed to crystallise. Suitably the reaction may be carried out at a temperature below ambient, e.g. 0 to 15° C, e.g. 0 to 10°C, suitably 0 to 0.5°C. Suitably if the salt of clavulanic acid with the polyamine of the general formula (II) is formed in aqueous solution it may be precipitated by admixing the aqueous solution with excess acetone.
Suitable methods of esterification include:
a)the reaction of the salt of clavulanic acid with the polyamine of the general formula (II) with a compound of the general formula Q-R¹¹, wherein Q is a readily displaceable group and R¹¹ is an organic group;
b)the reaction of the salt of clavulanic acid with the polyamine of the general formula (II) with an alcohol or thiol in the presence of a condensation promoting agent such as a carbodiimide; and
c) the reaction of the salt of clavulanic acid with the polyamine of the general formula (II) with a diazo compound.

The foregoing processes extend to cover those aspects wherein the salt of clavulanic acid with the polyamine of the general formula (II) is first converted to clavulanic acid or another salt thereof and subsequently is converted to the desired ester. Further details of esterification methods are disclosed in GB 1508977 and 1508978. The process of the present invention permits to obtain salts and esters of clavulanic acid more readily in pure form than by operation of the processes of GB1508977 and 1543563.

The invention will now be described by way of example only.

### Example

An impure solution of clavulanic acid in ethyl acetate (1L, 10.14 µg/ml) was prepared, obtained by extraction of an *S*. *clavuligerus* fermentation broth with ethyl acetate and some pre-purification by ion-exchange.

An impure wet (ca. 1% water) solution of clavulanic acid in ethyl acetate was obtained. 1L batches of this solution were added with stirring with an excess of ethylene diamine or N,N'-diethylethylene diamine or N,N'-diisopropylethylenediamine, the quantity of each amine being in excess of the amount needed to form the diammonium salt with the clavulanic acid. After continued stirring a precipitate of the salt formed. A further crop of crystals was obtained by addition of excess acetone. The precipitated diammonium salt was filtered off and washed with acetone.

The crystals of each of these diammonium salts so formed were converted to potassium clavulanate by dissolution of the salt in the minimum quantity of water, followed by the addition of a solution of an excess of potassium 2-ethylhexanoate in isopropyl alcohol. After continued stirring a precipitate of potassium clavulanate formed and was filtered, washed with isopropyl alcohol and dried.

## Claims (Claims for the following Contracting State(s): AT, CH, DE, DK, ES, FR, IT, LI, LU, NL, SE)

1. A process for the preparation and/or purification of clavulanic acid or a pharmaceutically acceptable salt or ester thereof which process comprises
i)
a) obtaining impure clavulanic acid in a broth resulting from fermentation of a clavulanic acid-producing microorganism,
b) at least removing some of the suspended solids in the broth by filtration,
c) contacting the obtained impure clavulanic acid in solution in an organic solvent with an amine of the general formula (II): 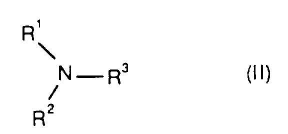
wherein R¹ is a group of the general formula 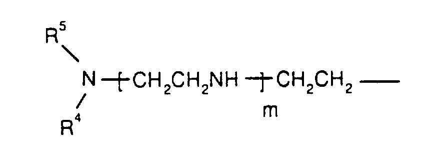 wherein R⁴ and R⁵ are independently hydrogen, C₁₋₆-alkyl, amino-substituted C₁₋₆-alkyl or substituted amino-substituted C₁₋₆-alkyl, and R and R³ are independently selected from hydrogen, C₁₋₆-alkyl, amino- or hydroxy-substituted C₁₋₆-alkyl or C₁₋₆-alkyl-substituted amino-substituted C₁₋₆-alkyl, and m is zero or an integer of 1 to 5;
ii) isolating the amine salt of clavulanic acid formed;
iii) converting the thus formed salt into clavulanic acid or a pharmaceutically acceptable salt or ester thereof.

2. A process according to claim 1 in which the broth of the impure clavulanic acid after having at least removed some of the suspended solids is preconcentrated.

3. A process according to claim 2 in which preconcentration is to a concentration of 10-100 mg/ml.

4. A process according to claim 2 in which the preconcentration is to a concentration of 10-40 mg/ml.

5. A process according to claim 2 in which the preconcentration is to a concentration of 10-25 g/L .

6. A process according to any one of claims 2 to 5 in which the preconcentration process is by absorption of the clavulanic acid onto an anion-exchange resin followed by elution of the clavulanic acid therefrom with an aqueous solution of an electrolyte.

7. A process according to claim 6 in which the electrolyte is sodium chloride.

8. A process according to claim 6 or 7 in which the eluent is de-salted.

9. A process according to any one of claims 1 to 8 in which one of R⁴ and R⁵ is hydrogen and the other is C₁₋₆-alkyl and R and R³ is hydrogen or C₁₋₆-alkyl.

## Claims (Claims for the following Contracting State(s): BE, GR, IE, MC, PT)

1. A process for the preparation and/or purification of clavulanic acid or a pharmaceutically acceptable salt or ester thereof which process comprises
i) contacting impure clavulanic acid in solution in an organic solvent with an amine of the general formula (II) 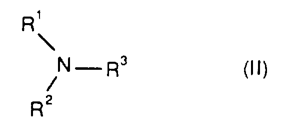 wherein R¹ is a group of the general formula 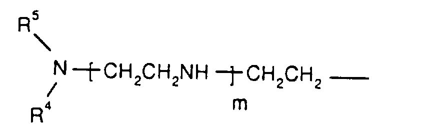 wherein R⁴ and R⁵ are independently hydrogen, C₁₋₆-alkyl, amino-substituted C₁₋₆-alkyl or substituted amino-substituted C₁₋₆-alkyl, and R and R³ are independently selected from hydrogen, C₁₋₆-alkyl, amino- or hydroxy-substituted C₁₋₆-alkyl or C₁₋₆-alkyl-substituted amino-substituted C₁₋₆-alkyl, and m is zero or an integer of 1 to 5;
ii) isolating the amine salt of clavulanic acid formed;
iii) converting the thus formed salt into clavulanic acid or a pharmaceutically acceptable salt or ester thereof.

2. A process for the preparation and/or purification of clavulanic acid or a pharmaceutically acceptable salt or ester thereof which process comprises
i)
a) obtaining impure clavulanic acid in a broth resulting from fermentation of a clavulanic acid-producing microorganism,
b) at least removing some of the suspended solids,
c) contacting the obtained impure clavulanic acid in solution in an organic solvent with an amine of the general formula (II): 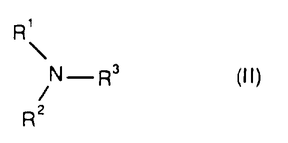 as defined in claim 1;
ii) isolating the amine salt of clavulanic acid formed;
iii) converting the thus formed salt into clavulanic acid or a pharmaceutically acceptable salt or ester thereof.

3. A process according to claim 2 in which in step i) b) the suspended solids are removed by filtration.

4. A process according to claim 2 in which the broth of the impure clavulanic acid after having at least removed some of the suspended solids is preconcentrated.

5. A process according to claim 4 in which the aqueous solution of clavulanic acid is preconcentrated to a concentration of 10-100 mg/ml.

6. A process according to claim 4 in which the aqueous solution of clavulanic acid is preconcentrated to a concentration of 10-40 mg/ml.

7. A process according to claim 4 in which the aqueous solution of clavulanic acid is preconcentrated to a concentration of 10-25 g/L.

8. A process according to any one of claims 4 to 7 in which the preconcentration process is by absorption of the clavulanic acid onto an anion-exchange resin followed by elution of the clavulanic acid therefrom with an aqueous solution of an electrolyte.

9. A process according to claim 8 in which the electrolyte is sodium chloride.

10. A process according to claim 8 or 9 in which the eluent is de-salted.

11. A process according to any one of claims 1 to 10 in which one of R⁴ and R⁵ is hydrogen and the other is C₁₋₆-alkyl and R and R³ is hydrogen or C₁₋₆-alkyl.

## Patentansprüche (Patentansprüche für folgende(n) Vertragsstaat(en): AT, CH, DE, DK, ES, FR, IT, LI, LU, NL, SE)

1. Verfahren zur Herstellung und/oder Reinigung von Clavulansäure oder eines pharmazeutisch verträglichen Salzes oder Esters davon, wobei das Verfahren umfaßt
i)
a) Erhalten unreiner Clavulansäure in einer Brühe, erhalten durch Fermentation eines Clavulansäure erzeugenden Mikroorganismus',
b) Entfernen mindestens einiger der in der Brühe suspendierten Feststoffe durch Filtration,
c) In-Kontakt-Bringen der erhaltenen unreinen Clavulansäure in Lösung in einem organischen Lösungsmittel mit einem Amin der allgemeinen Formel (II): 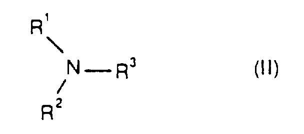
in der der Rest R¹ ein Rest der allgemeinen Formel 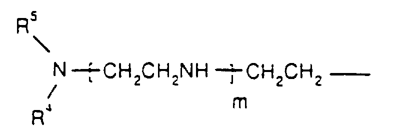 ist, wobei die Reste R⁴ und R⁵ unabhängig Wasserstoffatome, C₁₋₆-Alkyl-, aminosubstituierte C₁₋₆-Alkyl- oder substituierte aminosubstituierte C₁₋₆-Alkylreste sind und die Reste R und R³ unabhängig voneinander gewählt werden aus einem Wasserstoffatom, einem C₁₋₆-Alkyl-, einem amino- oder hydroxysubstituierten C₁₋₆-Alkyl- oder einem C₁₋₆-alkylsubstituierten aminosubstituierten C₁₋₆-Alkylrest und m den Wert null hat oder eine ganze Zahl von 1 bis 5 ist;
ii) Isolieren des gebildeten Amin-Salzes der Clavulansäure,
iii) Umwandeln des so gebildeten Salzes in Clavulansäure oder ein pharmazeutisch verträgliches Salz oder einem Ester davon.

2. Verfahren nach Anspruch 1, wobei die Brühe der unreinen Clavulansäure nach dem Entfernen mindestens einiger der suspendierten Feststoffe vorkonzentriert wird.

3. Verfahren nach Anspruch 2, wobei auf eine Konzentration von 10-100 mg/ml vorkonzentriert wird.

4. Verfahren nach Anspruch 2, wobei auf eine Konzentration von 10-40 mg/ml vorkonzentriert wird.

5. Verfahren nach Anspruch 2, wobei auf eine Konzentration von 10-25 g/l vorkonzentriert wird.

6. Verfahren nach einem der Ansprüche 2 bis 5, wobei das Vorkonzentrieren durch Adsorption der Clavulansäure an einem Anionenaustauscherharz, gefolgt von der Elution der Clavulansäure davon mit einer wäßrigen Lösung eines Elektrolyten erfolgt.

7. Verfahren nach Anspruch 6, wobei als Elektrolyt Natriumchlorid verwendet wird.

8. Verfahren nach Anspruch 6 oder 7, wobei das Eluat entsalzt wird.

9. Verfahren nach einem der Ansprüche 1 bis 8, wobei einer der Reste R⁴ und R⁵ ein Wasserstoffatom und der andere ein C₁₋₆-Alkylrest ist und die Reste R und R³ Wasserstoffatome oder C₁₋₆-Alkylreste sind.

## Patentansprüche (Patentansprüche für folgende(n) Vertragsstaat(en): BE, GR, IE, MC, PT)

1. Verfahren zur Herstellung und/oder Reinigung von Clavulansäure oder eines pharmazeutisch verträglichen Salzes oder Esters davon, wobei das Verfahren umfaßt
i) In-Kontakt-Bringen der unreinen Clavulansäure in Lösung in einem organischen Lösungsmittel mit einem Amin der allgemeinen Formel (II): 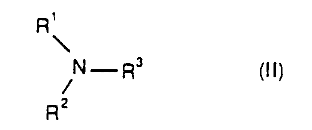 in der der Rest R¹ ein Rest der allgemeinen Formel 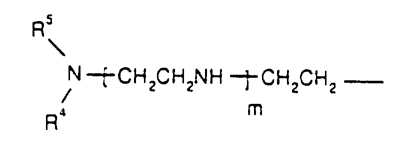 ist, wobei die Reste R⁴ und R⁵ unabhängig Wasserstoffatome, C₁₋₆-Alkyl-, aminosubstituierte C₁₋₆-Alkyl- oder substituierte aminosubstituierte C₁₋₆-Alkylreste sind und die Reste R und R³ unabhängig voneinander gewählt werden aus einem Wasserstoffatom, einem C₁₋₆-Alkyl-, einem amino- oder hydroxysubstituierten C₁₋₆-Alkyl- oder einem C₁₋₆-alkylsubstituierten aminosubstituierten C₁₋₆-Alkylrest und m den Wert null hat oder eine ganze Zahl von 1 bis 5 ist;
ii) Isolieren des gebildeten Amin-Salzes der Clavulansäure;
iii) Umwandeln des so gebildeten Salzes in Clavulansäure oder ein pharmazeutisch verträgliches Salz oder einem Ester davon.

2. Verfahren zur Herstellung und/oder Reinigung von Clavulansäure oder eines pharmazeutisch verträglichen Salzes oder Esters davon, wobei das Verfahren umfaßt
i)
a) Erhalten unreiner Clavulansäure in einer Brühe, erhalten durch Fermentation eines Clavulansäure erzeugenden Mikroorganismus',
b) Entfernen mindestens einiger der suspendierten Feststoffe,
c) In-Kontakt-Bringen der erhaltenen unreinen Clavulansäure in Lösung in einem organischen Lösungsmittel mit einem Amin der allgemeinen Formel (II): 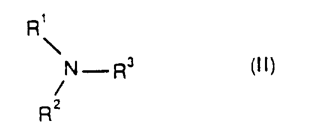
wie in Anpruch 1 definiert;
ii) Isolieren des gebildeten Aminsalzes der Clavulansäure;
iii) Umwandeln des so gebildeten Salzes in Clavulansäure oder ein pharmazeutisch verträgliches Salz oder einem Ester davon.

3. Verfahren nach Anspruch 2, wobei in Schritt i) b) die suspendierten Feststoffe durch Filtration entfernt werden.

4. Verfahren nach Anspruch 2, wobei die Brühe der unreinen Clavulansäure nach dem Entfernen mindestens einiger der suspendierten Feststoffe vorkonzentriert wird.

5. Verfahren nach Anspruch 4, wobei die wäßrige Lösung von Clavulansäure auf eine Konzentration von 10-100 mg/ml vorkonzentriert wird.

6. Verfahren nach Anspruch 4, wobei die wäßrige Lösung von Clavulansäure auf eine Konzentration von 10-40 mg/ml vorkonzentriert wird.

7. Verfahren nach Anspruch 4, wobei die wäßrige Lösung von Clavulansäure auf eine Konzentration von 10-25 g/l vorkonzentriert wird.

8. Verfahren nach einem der Ansprüche 4 bis 7, wobei das Vorkonzentrieren durch Adsorption der Clavulansäure an einem Anionenaustauscherharz, gefolgt von der Elution der Clavulansäure davon mit einer wäßrigen Lösung eines Elektrolyten erfolgt.

9. Verfahren nach Anspruch 8, wobei als Elektrolyt Natriumchlorid verwendet wird.

10. Verfahren nach Anspruch 8 oder 9, wobei das Eluat entsalzt wird.

11. Verfahren nach einem der Ansprüche 1 bis 10, wobei einer der Reste R⁴ und R⁵ ein Wasserstoffatom und der andere ein C₁₋₆-Alkylrest ist und die Reste R und R³ Wasserstoffatome oder C₁₋₆-Alkylreste sind.

## Revendications (Revendications pour l'(les) Etat(s) contractant(s) suivant(s): AT, CH, DE, DK, ES, FR, IT, LI, LU, NL, SE)

1. Procédé pour la préparation et/ou la purification de l'acide clavulanique ou d'un sel ou d'un ester pharmaceutiquement acceptable de celui-ci, lequel procédé comprend :
(i)
a) l'obtention d'acide clavulanique brut dans le bouillon résultant de la fermentation d'un micro-organisme producteur d'acide clavulanique,
b) l'élimination d'au moins quelques unes des particules solides en suspension dans le bouillon par filtration,
c) la mise en contact de l'acide clavulanique brut obtenu, en solution dans un solvant organique, avec une amine de formule générale (II) : 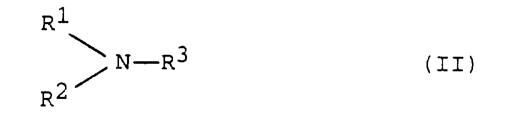 dans laquelle R¹ est un groupe de formule générale : 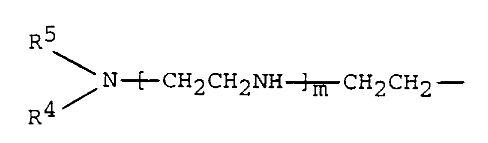 dans laquelle R⁴ et R⁵ sont indépendament un atome d'hydrogène, un groupe alkyle en C₁-C₆, un groupe alkyle en C₁-C₆ substitué par une amine ou un groupe alkyle en C₁-C₆ substitué par une amine substituée, R et R³ sont indépendament choisis parmi un atome d'hydrogène, un groupe alkyle en C₁-C₆, un groupe alkyle en C₁-C₆ substitué par une amine ou par un groupe hydroxyle et un groupe alkyle en C₁-C₆ substitué par une amine substituée par un groupe alkyle en C₁-C₆, m est égal à 0 ou est un nombre entier compris entre 1 à 5 ;
ii) l'isolation du sel aminé d'acide clavulanique formé ;
iii) la conversion du sel ainsi formé en acide clavulanique ou en un sel ou un ester de celui-ci pharmaceutiquement acceptable.

2. Procédé selon la Revendication 1 dans lequel le bouillon d'acide clavulanique brut est préconcentré après avoir éliminé au moins quelques unes des particules solides en suspension.

3. Procédé selon la Revendication 2 dans lequel la préconcentration correspond à une concentration de 10-100 mg/ml.

4. Procédé selon la Revendication 2 dans lequel la préconcentration correspond à une concentration de 10-40 mg/ml.

5. Procédé selon la Revendication 2 dans lequel la préconcentration correspond à une concentration de 10-25 g/l.

6. Procédé selon l'une quelconque des Revendications 2 à 5 dans lequel la préconcentration se fait par absorption de l'acide clavulanique sur une résine échangeuse d'anions suivie de l'élution de l'acide clavulanique avec une solution aqueuse d'un électrolyte.

7. Procédé selon la Revendication 6 dans lequel l'électrolyte est du chlorure de sodium.

8. Procédé selon la Revendication 6 ou la Revendication 7 dans lequel l'éluant est désalifié.

9. Procédé selon l'une quelconque des Revendications 1 à 8 dans lequel l'un des radicaux R⁴ et R⁵ est un atome d'hydrogène et l'autre radical est un groupe alkyle en C₁-C₆, et R et R³ sont un atome d'hydrogène ou un groupe alkyle en C₁-C₆.

## Revendications (Revendications pour l'(les) Etat(s) contractant(s) suivant(s): BE, GR, IE, MC, PT)

1. Procédé pour la préparation et/ou la purification de l'acide clavulanique ou d'un sel ou d'un ester pharmaceutiquement acceptable de celui-ci, lequel procédé comprend :
(i) la mise en contact de l'acide clavulanique brut en solution dans un solvant organique avec une amine de formule générale (II) : 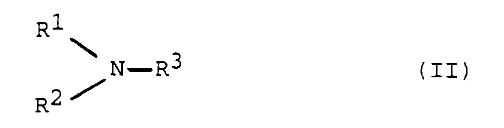 dans laquelle R¹ est un groupe de formule générale : 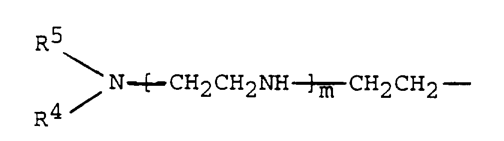 dans laquelle R⁴ et R⁵ sont indépendament un atome d'hydrogène, un groupe alkyle en C₁-C₆, un groupe alkyle en C₁-C₆ substitué par une amine ou un groupe alkyle en C₁-C₆ substitué par une amine substituée, R et R³ sont indépendament choisis parmi un atome d'hydrogène, un groupe alkyle en C₁-C₆, un groupe alkyle en C₁-C₆ substitué par une amine ou par un groupe hydroxyle et un groupe alkyle en C₁-C₆ substitué par une amine substituée par un groupe alkyle en C₁-C₆, m est égal à 0 ou est un nombre entier compris entre 1 et 5 ;
ii) l'isolation du sel aminé d'acide clavulanique formé ;
iii) la conversion du sel ainsi formé en acide clavulanique ou en un sel ou un ester de celui-ci pharmaceutiquement acceptable.

2. Procédé pour la préparation et/ou la purification de l'acide clavulanique ou d'un sel ou d'un ester pharmaceutiquement acceptable de celui-ci, lequel procédé comprend :
(i)
a) l'obtention d'acide clavulanique brut dans le bouillon résultant de la fermentation d'un micro-organisme producteur d'acide clavulanique,
b) l'élimination d'au moins quelques unes des particules solides en suspension,
c) la mise en contact de l'acide clavulanique brut obtenu, en solution dans un solvant organique, avec une amine de formule générale (II) : 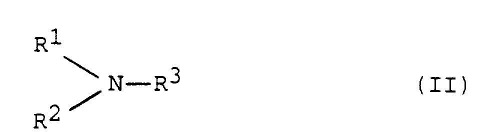 telle que définie dans la Revendication 1 ;
ii) l'isolation du sel aminé d'acide clavulanique formé ;
iii) la conversion du sel ainsi formé en acide clavulanique ou en un sel ou un ester de celui-ci pharmaceutiquement acceptable.

3. Procédé selon la Revendication 2 dans lequel, à l'étape i) b), les particules solides en suspension sont éliminées par filtration.

4. Procédé selon la Revendication 2 dans lequel le bouillon d'acide clavulanique brut est préconcentré après avoir éliminé au moins quelques unes des particules solides en suspension.

5. Procédé selon la Revendication 4 dans lequel la solution aqueuse d'acide clavulanique est préconcentrée à une concentration de 10-100 mg/ml.

6. Procédé selon la Revendication 4 dans lequel la solution aqueuse d'acide clavulanique est préconcentrée à une concentration de 10-40 mg/ml.

7. Procédé selon la Revendication 4 dans lequel la solution aqueuse d'acide clavulanique est préconcentrée à une concentration de 10-25 g/l.

8. Procédé selon l'une quelconque des Revendications 4 à 7 dans lequel la préconcentration se fait par absorption de l'acide clavulanique sur une résine échangeuse d'anions suivie de l'élution de l'acide clavulanique avec une solution aqueuse d'un électrolyte.

9. Procédé selon la Revendication 8 dans lequel l'électrolyte est du chlorure de sodium.

10. Procédé selon la Revendication 8 ou la Revendication 9 dans lequel l'éluant est désalifié.

11. Procédé selon l'une quelconque des Revendications 1 à 10 dans lequel l'un des radicaux R⁴ et R⁵ est un atome d'hydrogène et l'autre radical est un groupe alkyle en C₁-C₆ et R et R³ sont un atome d'hydrogène ou un groupe alkyle en C₁-C₆.
